**Europäisches Patentamt**

**European Patent Office**

**Office européen des brevets**

(11) Veröffentlichungsnummer: **0 263 938 B1**

# EUROPÄISCHE PATENTSCHRIFT

(45) Veröffentlichungstag der Patentschrift: **06.11.91**

(51) Int. Cl.⁵: **A61B 17/58**

(21) Anmeldenummer: **87111511.9**

(22) Anmeldetag: **08.08.87**

(54) **Zugschraube fuer die Osteosynthese.**

(30) Priorität: **19.08.86 AT 2221/86**

(43) Veröffentlichungstag der Anmeldung:
**20.04.88 Patentblatt 88/16**

(45) Bekanntmachung des Hinweises auf die
Patenterteilung:
**06.11.91 Patentblatt 91/45**

(84) Benannte Vertragsstaaten:
**BE CH DE ES FR GB IT LI SE**

(56) Entgegenhaltungen:
**EP-A- 0 016 338**
**FR-A- 2 275 679**
**GB-A- 1 489 684**
**US-A- 2 490 364**

(73) Patentinhaber: **Krenkel, Christian**
**Moosstrasse 126**
**A-5020 Salzburg(AT)**

Patentinhaber: **Lixl, Georg**
**Untereching 72**
**A-5110 Oberndorf(AT)**

(72) Erfinder: **Krenkel, Christian**
**Moosstrasse 126**
**A-5020 Salzburg(AT)**
Erfinder: **Lixl, Georg**
**Untereching 72**
**A-5110 Oberndorf(AT)**

(74) Vertreter: **Hofinger, Engelbert et al**
**Torggler-Hofinger Wilhelm-Greil-Strasse 16**
**A-6020 Innsbruck(AT)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die Erfindung betrifft eine Zugschraube für die Osteosynthese mit einem Senkkopf und einer den Schaft der Zugschraube umgebenden Unterlegscheibe, die eine dem Senkkopf entsprechende, obere Einsenkung und schraubenschaftseitig eine eine umlaufende Kante bildende, untere Einsenkung aufweist.

Um bei Knochenfrakturen oder in der plastischen Chirurgie die Knochenflächen für die Osteosynthese zusammenzufügen, werden u.a. Zugschrauben verwendet. Die Zugschrauben werden möglichst senkrecht zu der Bruchfläche eingesetzt und werden vorzugsweise als selbstschneidende Schraube in ein Zugloch des anderen Knochenfragmentes eingeschraubt.

Üblicherweise werden heute Zugschrauben mit - vorzugsweise kugeligem Senkkopf (vgl. DE-A-27 47 312) ohne Unterlegscheibe verwendet.

Da die Zugschrauben möglichst senkrecht zur Bruchfläche des Knochens eingesetzt werden müssen, ergibt sich in der Regel, daß die Zugschraube mehr oder weniger schräg zur Knochenoberfläche eingesetzt werden muß. Dies hat bei den bekannten Zugschrauben zur Folge, daß in dem Moment, in welchem der Schraubenkopf bzw. die Unterlegscheibe mit der Knochenoberfläche, der Substantia corticalis, in Berührung kommt, ein Kraftmoment entsteht, welches den Schraubenkopf aus der Schraubenachsenrichtung nach außen abdrängt. Dadurch kann die Schraube sogar verbogen werden. Insbesondere kann das auf den Schraubenkopf wirkende Kippmoment zu einer Verschiebung der Bruchflächen führen. Um ein solches durch die einseitige Berührung des Schraubenkopfes bzw. der Unterlegscheibe an dem Knochen verursachtes Kippmoment zu vermeiden, ist es üblich, in dem Knochen eine Senkung für den Schraubenkopf vorzusehen, die so tief durchgeführt wird, daß der Schraubenkopf allseitig gleichmäßig mit dem Knochen in Berührung kommt.

Dies hat schwerwiegende Nachteile zur Folge. Bei vielen Knochen ist die harte äußere Substantia corticalis nur dünnwandig, so daß die Gefahr besteht, daß der Schraubenkopf nach innen in die wenig widerstandsfähige Substantia spongiosa abrutscht. Der Senkkopf der Schraube kann in der konkaven Einsenkung des Knochens eine Keilwirkung erzeugen, die einen vom Bohrloch ausgehenden Bruch verursachen kann, insbesondere wenn mehrere Zugschrauben nebeneinandergesetzt werden müssen. Der Druck auf das bruchspaltnahe Knochenfragment erzeugt auf den zwischen den Bohrlöchern liegenden Knochen eine Zugwirkung, die zu einem Absprengen des Fragmentendes führen kann. Die Einfräsung für den Schraubenkopf muß für einen allseitigen Kontakt des Schraubenkopfes mit dem Knochen, insbesondere bei schrägem Eintrittswinkel, sehr tief sein, wodurch sich die Einfräsung dem Bruchspalt nähert. Es wird dadurch weniger Knochensubstanz für die Osteosynthese durch die Schraube erfaßt. Die tiefe Einfräsung für den Schraubenkopf hat außerdem zur Folge, daß der Schraubenkopf in den Knochen einwächst und zur Entfernung der Schraube erst freigefräst werden muß. Ist die Einfräsung für den Schraubenkopf nicht exakt und weist eine leichte Abwinkelung gegenüber der Achse des Gleitloches auf, so wandert der Schraubenkopf beim Festziehen, was ebenso zu unerwünschten punktförmigen Spannungen und einem Kippmoment auf die Schraubenachse führen kann, das die reponierte Fraktur wieder verschieben kann. Alle diese Nachteile begrenzen die Einsatzmöglichkeiten von Zugschrauben in bezug auf den Winkel der Schraube zur Bruchfläche und zur Knochenoberfläche.

Der Erfindung liegt die Aufgabe zugrunde, eine Zugschraube für die Osteosynthese zur Verfügung zu stellen, die diese Nachteile weitgehend vermeidet oder zumindest verringert und auch bei dünnwandiger Substantia corticalis und beim schrägem Eintrittswinkel in den Knochen eingesetzt werden kann.

Die gestellte Aufgabe wird keineswegs bereits dadurch gelöst, daß die Zugschraube mit irgend einer Unterlegscheibe versehen wird. Beispielsweise zeigt US-A-2 490 364 eine Einrichtung der eingangs definierten Gattung, bei welcher die verwendete Unterlegscheibe einen größeren Durchmesser aufweist als die Schraube. Dadurch wird bei sattem Aufliegen der Unterlegscheibe auf dem Knochen sichergestellt, daß sich beim Anziehen der Schraube deren Kopf in der Beilagscheibe dreht und nicht die Beilagscheibe im Knochen. Bei Schrägstellung der Schraube würde das Problem der achsfernen Einleitung von Kräften durch die Unterlegscheibe hingegen noch verschärft. Die Erfindung löst die gestellte Aufgabe demgegenüber dadurch, daß die Unterlegscheibe als zylindrischer Teil ausgebildet ist, dessen Außendurchmesser im wesentlichen dem des Senkkopfes entspricht, und daß die Kante derart keilförmig ausgebildet ist, daß sie sich bei Eindrehung der Zugschraube in einen Knochen in dessen Substantia corticalis einkrallt.

Aus GB-A-1 489 684 ist zwar bereits eine Unterlegscheibe bekanntgeworden, deren Kantenform in etwa dem hier gemachten Vorschlag entspricht. Die Oberseite dieser Unterlegscheibe ist jedoch konvex ausgebildet, um in die konkave Unterseite der Zugschraube zu passen. Eine solche Schraubenform ist auf die Anwendung bei großen Knochen beschränkt. Bei einer Dimensionierung, wie sie für Operationen im Bereich des Gesichtsschädels notwendig ist, würde eine derart ohne Senkkopf ausgebildete Schraube nicht die erforderliche Festigkeit aufweisen.

Die Unterlegscheibe verkrallt sich aufgrund ihrer schraubenschaftseitigen keilförmigen Kante verrückungsfest an jeder Stelle, auch wenn sie bei schrägem Einsetzwinkel der Schraube nur über die Hälfte ihres Außenumfangs mit dem Knochen in Berührung steht. Ein auf die Schraubenachse wirkendes kippendes Kraftmoment wird durch die kegelförmige Einsenkung der Unterlegscheibe vermieden und die Kräfte werden auf eine größere Fläche verteilt.

Vorzugsweise weist die kegelförmige, kugelsegmentförmige oder sphärische Einsenkung einen Scheitelwinkel von etwa 120° auf, so daß die schraubenschaftseitige Kante der Unterlegscheibe einen Winkel von etwa 30° - 60° aufweist. Dies ergibt eine günstige Abstützwirkung, ohne daß die Kante der Unterlegscheibe zu stark in die Knochensubstanz einschneidet.

Durch die erfindungsgemäße Unterlegscheibe kann eine Zugschraube unter sehr flachem Winkel zur Knochenoberfläche eingesetzt werden, ohne abzurutschen. Dies erweitert die Einsatzmöglichkeiten der Zugschraube für die Osteosyntese erheblich.

Die Zugschraubenkräfte setzen im Gegensatz zur herkömmlichen Zugschraube nicht nach außen in Richtung auf den Bruchspalt an, sondern werden durch die Unterlegscheibe nach innen von dem Bruchspalt und dem Bohrloch weggeleitet. Die Drucklinien verlaufen dadurch mehr in der Nähe der Knochenachse und es wird ein wesentlich größerer Anteil des Knochens für die Osteosynthese herangezogen.

Da es ausreicht, daß die Unterlegscheibe mit etwa der Hälfte ihres Umfanges mit der Knochenoberfläche in Kontakt kommt, muß eine wesentlich geringere Einsenkung in den Knochen eingefräst werden, so daß der Schraubenkopf weiter vom Bruchspalt wegbleibt, was für die Stabilität der Osteosynthese von Vorteil ist. Außerdem bleibt der Schraubenkopf außerhalb der Knochenoberfläche, so daß die Schrauben leichter zu entfernen sind.

Bei sehr schräger Eintrittsrichtung der Zugschraube wird der Zug der Schraube über die Unterlegscheibe in eine Zugkraft in die Knochenregion verlagert, die vom Schraubenloch und Frakturspalt ferne liegt. Einem Absprengen des Fragmentendes wird dadurch vorgebeugt.

Die erfindungsgemäße Zugschraube kann nicht nur in allen Anwendungsfällen der herkömmlichen Zugschrauben eingesetzt werden, sondern eignet sich darüber hinaus auch für alle Schrägfrakturen der Extremitätenknochen, wofür bisher Circlagen oder Platten eingesetzt werden mußten, für die Fingerchirurgie und für knöcherne Sehnenausrisse. Auch Frakturen, die verhältnismäßig senkrecht zur Knochenoberfläche verlaufen und bisher die Verwendung von Platten erforderten, können mit der erfindungsgemäßen Zugschraube verbunden werden, aufgrund der durch die Unterlegscheibe gegebenen Möglichkeit, die Zugschraube sehr schräg zur Knochenoberfläche einzusetzen. Dies ist insbesondere für die Kieferchirurgie von großer Wichtigkeit.

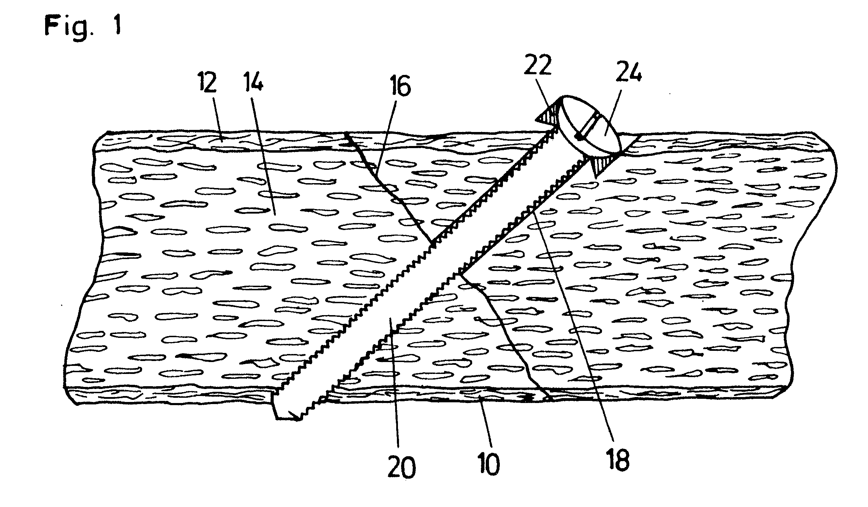

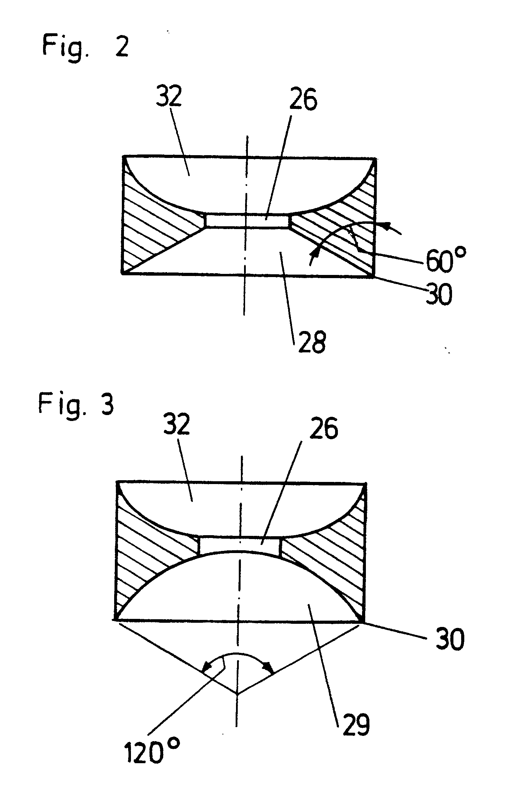

Im folgenden wird die Erfindung anhand eines in der Zeichnung dargestellten Ausführungsbeispieles näher erläutert. Es zeigen
Fig. 1 einen Knochen mit eingesetzter Zugschraube im Axialschnitt und Fig. 2 und 3 vergrößert zwei Ausführungsformen der Unterlegscheibe der Zugschraube.

In Fig. 1 ist im Axialschnitt ein Knochen 10 mit der äußeren Substantia corticalis 12 und der inneren Substantia spongiosa 14 dargestellt. Der Knochen 10 weist eine Fraktur mit dem Bruchspalt 16 auf.

Für die Osteosynthese der Fraktur ist ein Gleitloch 18 in das eine Knochenfragment bis zu dem Bruchspalt 16 gebohrt. Das Gleitloch 18 verläuft schräg zur Knochenachse und möglichst senkrecht zu dem Bruchspalt 16.

Eine Zugschraube 20 mit selbstschneidendem Gewinde ist in das Gleitloch 18 eingeschoben und in eine engere Vorbohrung des anderen Bruchfragmentes selbstschneidend eingedreht.

Die Zugschraube 20 weist eine in Fig. 2 und 3 vergrößert dargestellte Unterlegscheibe 22 und einen Senkkopf 24 auf.

Die Unterlegscheibe 22 nach Fig. 2 ist ein zylindrisches Teil mit einem Außendurchmesser, der im wesentlichen dem Außendurchmesser des Senkkopfes 24 entspricht. Die Unterlegscheibe 22 weist eine axiale Bohrung 26 auf. Schraubenschaftseitig ist die Bohrung 26 durch eine kegelförmige Einsenkung 28 erweitert. Die Einsenkung 28 erweitert sich bis an den Außenumfang der Unterlegscheibe 22, so daß eine ringsum laufende keilförmige Kante 30 ausgebildet wird. Die Einsenkung 28 weist einen Scheitelwinkel von 120° auf, so daß sich ein Keilwinkel der Kante 30 von 60° ergibt.

Schraubenkopfseitig weist die Unterlegscheibe 22 eine Einsenkung 32 in Form eines Kugelsegmentes auf, in welche sich der kugelsegmentförmige Senkkopf 24 einfügt.

Die Unterlegscheibe nach Fig. 3 weist eine kugelsegmentförmige bzw. sphärische Einsenkung 29 auf. Die Einsenkung 29 weist einen Scheitelwinkel des Kugelsegmentes von ca. 120° auf, so daß sich ein Keilwinkel der Kante 30 von ca. 30° ergibt.

## Patentansprüche

1. Zugschraube (20) für die Osteosynthese mit einem Senkkopf (24) und einer den Schaft der Zugschraube (20) umgebenden Unterlegscheibe (22), die eine dem Senkkopf (24) entsprechende obere Einsenkung (32) und schraubenschaftseitig eine eine umlaufende Kante (30) bildende untere Einsenkung (28,29) aufweist, dadurch gekennzeichnet, daß die Unterlegscheibe (22) als zylindrischer Teil ausgebildet ist, dessen Außendurchmesser im wesentlichen dem des Senkkopfes (24) entspricht, und daß die Kante (30) derart keilförmig ausgebildet ist, daß sie sich bei Eindrehung der Zugschraube (20) in einen Knochen (10) in dessen Substantia corticalis einkrallt.

2. Zugschraube nach Anspruch 1, dadurch gekennzeichnet, daß die untere Einsenkung (29) kugelsegmentförmig ist und einen Kugelsegmentwinkel von etwa 120° aufweist.

3. Zugschraube nach Anspruch 1, dadurch gekennzeichnet, daß die untere Einsenkung (28) kegelförmig ist und einen Scheitelwinkel von etwa 120° aufweist.

## Claims

1. A pulling screw (20) for osteosynthesis comprising a countersunk head (24) and a support washer (22) which is disposed around the shank of the pulling screw (20) and which has an upper countersink portion (32) corresponding to the countersunk head (24) and on the side towards the screw shank a lower countersink portion (28, 29) forming a peripherally extending edge (30), characterised in that the support washer (22) is in the form of a cylindrical member whose outside diameter substantially corresponds to that of the countersunk head (24) and that the edge (30) is of a wedge-shaped configuration in such a way that when the pulling screw (20) is screwed into a bone (10) the edge (30) digs into the Substantia corticalis of the bone.

2. A pulling screw according to claim 1 characterised in that the lower countersink portion (29) is in the shape of a segment of a sphere and has a spherical segment angle of about 120°.

3. A pulling screw according to claim 1 characterised in that the lower countersink portion (28) is conical and has an apex angle of about 120°.

## Revendications

1. Vis de traction (20) pour ostéosynthèse, comprenant une tête fraisée (24) et une rondelle (22) qui entoure la tige de la vis de traction (20), et qui présente un fraisage supérieur (32) correspondant à la tête fraisée (24) et, côté tige de vis, un fraisage inférieur (28, 29) formant une arête périphérique, caractérisée en ce que la rondelle (22) est constituée par une pièce cylindrique dont le diamètre extérieur correspond sensiblement à celui de la tête fraisée (24), et en ce que l'arête (30) est d'une configuration conique telle que, lorsqu'on visse la vis de traction (20), cette tête morde dans l'os (10), plus précisément dans sa substantia corticalis.

2. Vis de traction selon la revendication 1, caractérisée en ce que le fraisage inférieur (29) est en forme de segment sphérique et présente un angle de segment sphérique d'environ 120°.

3. Vis de traction selon la revendication 1, caractérisée en ce que le fraisage inférieur (28) est conique et présente un angle au sommet d'environ 120°.

Fig. 1

Fig. 2

Fig. 3